# EUROPEAN PATENT SPECIFICATION

(11) **EP 2 690 096 B1**
(45) Date of publication and mention of the grant of the patent: **26.11.2014**
(21) Application number: 13163191.3
(22) Date of filing: 10.04.2013
(51) Int. Cl.: C07D 235/16, A61K 31/4184, A61P 35/00

(54) **Process for preparation of Bendamustine**
Verfahren zur Herstellung von Bendamustin
Procédé de préparation de Bendamustine

(30) Priority: 24.07.2012 EP 12177730
(43) Date of publication of application: 29.01.2014
(73) Proprietor: HEYL Chemisch-Pharmazeutische Fabrik GmbH und Co. KG, 14167 Berlin (DE)
(72) Inventor: Frey, Michael, 99423 Weimar (DE); Walther, Dirk-Detlef, 99510 Apolda (DE)
(74) Representative: Junghans, Claas

(56) References cited:
- WO-A1-2010/042568
- WO-A2-2011/079193
- DD-A- 34 727

## Description

The present invention relates to a process for the preparation of 4-[5-[bis(2-chloroethyl)amino]-1-methyl-*1H-*benzimidazol-2-yl]butanoic acid (9, Bendamustine) starting from 2-fluoro-5-nitroaniline and proceeding through the intermediates 5-(2-fluoro-5-nitroanilino)-5-oxopentanoic acid (1) and 5-[2-(methylamino)-5-nitroanilino]-5-oxopentanoic acid (3).

Bendamustine belongs to the alkylating agent class of compounds, in particular to the nitrogen mustard derivatives. As an antitumoral chemotherapeutic agent it is used both for the treatment of hematological tumours such as non-Hodgkin's and Hodgkin's lymphoma, mantle cell lymphoma, chronic lymphocytic leukaemia or multiple melanoma, as well as for the treatment of solid tumours such as breast cancer or small cell lung cancer.

The compound was first described in 1963 by Ozegowski and Krebs (see Ozegowski W, Krebs D. Aminosäureantagonisten. III. ω-[Bis-(β-chloräthyl)-amino-benzimidazolyl-(2)]-propion- bzw. -buttersäuren als potentielle Cytostatika. J. Prakt. Chem., 1963 Jun; 20 (3-4): 178-186, and references cited therein) and was marketed under the name Cytostasan. In 1993, the compound was approved in Germany under the name Ribomustin. In 2008 the compound was approved by the FDA for the treatment of B-cell non-Hodgkin's lymphoma and chronic lymphocytic leukaemia under the trade name Treanda.

Numerous synthetic routes for the preparation of Bendamustine are known in the prior art.

Ozegowski *et al.* (1963) describe the synthesis of Bendamustine starting from N1-methyl-4-nitrobenzene-1,2-diamine.

The synthesis of Bendamustine starting from 1-methylamino-2,4-dinitrobenzene or 2,4-dinitroaniline is described in WO2010042568. The alkylation of the 1-methyl-5-aminobenzimidazole derivative to Bendamustine alkyl ester takes place directly to the crude Bendamustine alkylester with chloroacetic acid, chloroacetic acid ester or chloroacetaldehyde and subsequent reduction with borane-THF complex (reductive amination).

DD34727 describes a process for the preparation of N1-substituted derivatives of Bendamustine, wherein the alkylation of the 1-methyl-5-aminobenzimidazole derivative is performed with ethylene oxide.

WO2011079193 describes the alkylation of a 1-methyl-5-amino-benzimidazole derivative to the precursor of Bendamustine (bishydroxy compound) using 2-Haloethanols in the presence of an organic base.

An analogous alkylation of a 1-methyl-5-amino-benzimidazole derivative in the presence of inorganic bases such as sodium or potassium carbonate is described in WO2012007966 and IPCOM000185126D.

The known processes for the production of Bendamustine exhibit a number of disadvantages, such as the formation of by-products or low yields. In particular, the yield of the favoured procedure in WO2011079193 (using Hünig's base) is not greater than 44.5% and gives a purity of 97.6%.

Some reaction steps are not implementable in large scale or industrial processes. Often, ethylene oxide, a toxic and explosive gas, is used during the synthesis. The use of this gas is unfavourable for reasons of occupational safety.

The objective of the present invention is to provide means and methods that enable an economical preparation of Bendamustine whilst avoiding the disadvantages of the methods known in the art. This objective is attained by the subject matter of the independent claims.

According to one aspect of the invention, a method for the preparation of 4-(l-methyl-5-nitro-benzimidazol-2-yl)butanoic acid (4) starting from 2-fluoro-5-nitroaniline is provided. Therein, 2-fluoro-5-nitroaniline is converted to 5-(2-fluoro-5-nitroanilino)-5-oxopentanoic acid (1, CAS No 451459-95-3) using glutaric anhydride in a step a).

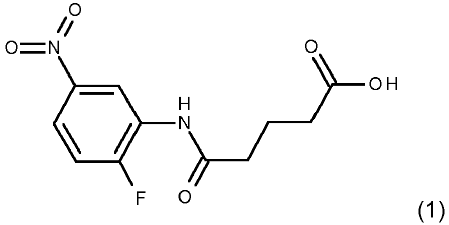

The reaction product of step a) is subsequently converted to methylammonium 5-[2-(methylamino)-5-nitroanilino]-5-oxopentanoate (2) using methylamine in a step b), which is in turn converted in a step c) to the corresponding acid, 5-[2-(methylamino)-5-nitroanilino]-5-oxopentanoic acid (3, CAS-Nr. 91644-13-2).

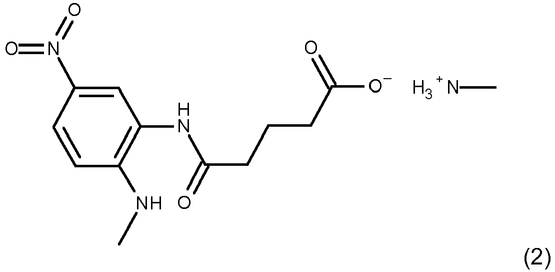

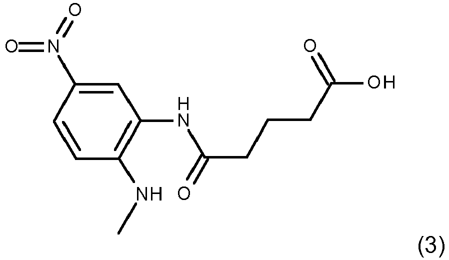

In a step d), the reaction product (3) of step c) is condensed to 4-(1-methyl-5-nitro-*1H-*benzimidazol-2-yl)butanoic acid (4, CAS-Nr. 31349-48-1).

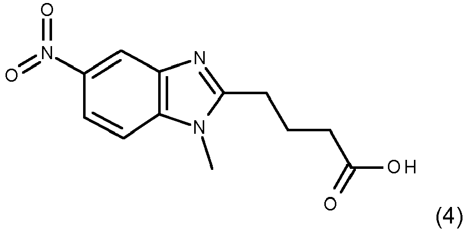

In one embodiment, the reaction steps a) to d) are performed in a single process step without isolation of the reaction products. The reactions can be performed in a one-pot laboratory process.

The use of glutaric anhydride in step a) is advantageous in comparison to the use of alternative reagents such as acid chlorides, as the formation of viscous by-products is reduced.

In one embodiment, the reaction of step a) is performed in a solvent that is chemically inert towards glutaric anhydride. Examples for such solvents include, without being restricted to, aliphatic, aromatic, cycloaliphatic or chlorinated hydrocarbons, ethers or carboxylic acid esters. Preferably, tetrahydrofuran or toluene is used.

In one embodiment of this aspect of the invention, the reaction of step b) is performed in an aqueous or alcoholic solution of methylamine, wherein the concentration of the aqueous or alcoholic methylamine solution is 25-40%, preferably 30 %.

In one embodiment, the product of step d), 4-(1-methyl-5-nitro-*1H*-benzimidazol-2-yl)butanoic acid (4), is isolated.

In one embodiment, the product of step d), 4-(1-methyl-5-nitro-*1H*-benzimidazol-2-yl)butanoic acid (4), is converted to alkyl 4-(1-methyl-5-nitro-*1H*-benzimidazol-2-yl)butanoate (5, CAS No 3543-72-4 (R=ethyl-)) in a step e), wherein R is CH₃ (methyl-), C₂H₅ (ethyl-), C₃H₇ (propyl-) or C₄H₉ (butyl-), generally CₙH₂ₙ₊₁.

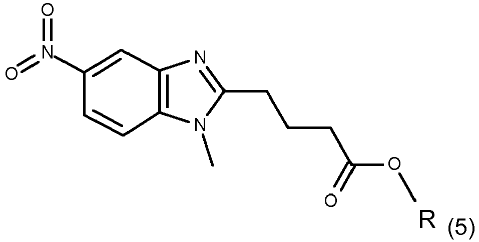

The reaction of step e) is performed with a primary or secondary C1 to C4 alcohol.

In one embodiment the reaction is performed with methanol, 1-propanol, 2-propanol, 1-butanol, 2-methyl-1-propanol or 2-butanol, preferably with ethanol.

In one embodiment, the reaction is performed in the presence of a strong acid. A strong acid in the sense of the present specification is defined by a pK_{A} value < 1.5. Preferably, sulfuric acid (H₂SO₄) is used.

In one embodiment, the amount of acid employed ranges from 10 - 90 % in relation to the amount of substance of the product of step d), 4-(1-methyl-5-nitro-1*H*-benzimidazol-2-yl)butanoic acid (4) used, preferably 60 - 75 %.

The process disclosed in DD 34727 uses more than 150 % acid based on the molar quantity of the product of step d) used. The yields that are achieved under these reaction conditions are thereby lower than those achieved by the method presented herein (76 % versus 88 %). Thus, compared with the prior art, the yields are significantly increased by 12 %.

In one embodiment, R is ethyl.

In a further embodiment of this aspect of the invention, the steps a) to e) are performed in a single process step without isolation of the reaction products.

Therein, the methylammonium 5-[(2-methylamino-5-nitrophenyl)amino]-5-oxopentanoate (2) is concentrated to dryness under vacuum. To remove residual water and further methylamine gas, the material is concentrated multiple times firstly from propanol and finally from ethanol to give a solid residue. Following addition of identical quantities of ethanol and acid as per an isolation according to step d), step e) is immediately instigated and the compound alkyl 4-(1-methyl-5-nitro-*1H*-benzimidazol-2-yl) butanoate (5, CAS No 3543-72-4 (R=ethyl-)) obtained in the same fashion. The advantage of this procedure is that a further step is added to the one-pot procedure.

Due to the formation of hydrogen fluoride during the reaction, the procedure must be carried out in an apparatus being inert toward hydrogen fluoride.

A further embodiment of the production of alkyl 4-(1-methyl-5-nitro-*1H*-benzimidazol-2-yl) butanoate (5, CAS No 3543-72-4 (R=ethyl-)) entails initially heating 2-fluoro-5-nitroaniline with aqueous or alcoholic methylamine solution at 80-90°C over several hours yielding compound N-1-methyl-4-nitrobenzene-1,2-diamine (CAS 41939-61-1), whereby the concentration of the methylamine solution is 25-40%, preferably 30%.

The resulting compound N-1-methyl-4-nitrobenzene-1,2-diamine (CAS 41939-61-1) is subsequently converted to 5-[(2-methylamino-5-nitrophenyl)amino]-5-oxopentanaoic acid (3, CAS-Nr. 91644-13-2) using glutaric acid anhydride in tetrahydrofuran. Complete concentration to dryness leaves compound (3).

Compound (3) is then converted to 4-(1-methyl-5-nitro-*1H*-benzimidazol-2-yl)butanoic acid - alkyl ester (5, CAS-Nr. 3543-72-4 (R = Ethyl-)) using ethanol and acid catalyst in the same ratio as with the compound 4-(1-methyl-5-nitro-*1H*-benzimidazol-2-yl)butanoic acid (4, CAS-Nr. 31349-48-1) in step e).

In comparison to the earlier described process, a challenge of this procedure is that the conversion to N-1-Methyl-4-nitrobenzene-1,2-diamine (CAS 41939-61-1) requires higher excesses and proceeds significantly slower (here > 10 hours compared to max. 2 hours). In addition, the compound to be isolated and dried is strongly electrostatically chargeable, which leads to substantial handling difficulties.

In one embodiment of the invention, a method for the preparation of alkyl 4-(5-amino-1-methyl-*1H*-benzimidazol-2-yl)butanoate (6, CAS No 3543-73-5 (R=ethyl-)) through selective reduction of compound (5) is provided, wherein R is CH₃ (methyl-), C₂H₅ (ethyl-), C₃H₇ (propyl-) or C₄H₉ (butyl-), generally CₙH₂ₙ₊₁.

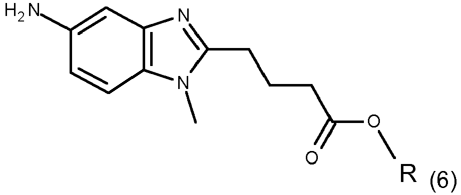

In one embodiment, the reduction is performed with hydrogen using a palladium catalyst doped with iron. The use of catalysts based on palladium doped with iron is advantageous in comparison to the use of catalysts based on platinum doped with vanadium, as the formation of intermediates or by-products is strongly suppressed and, consequently, the synthesis can be designed to be more economical. With the use of pure palladium catalysts increased levels of by-products occur towards the end of the hydrogenation which, through several recrytallisations with loss of yield, are only moderately removable.

In one embodiment, the palladium catalyst is doped with iron.

In one embodiment, an additional iron(II) or iron(III) salt of an organic or inorganic acid is added in catalytic amounts to the used catalyst.

In one embodiment, the additional iron (II) or iron (III) salt is selected from iron(II) sulfate-7-hydrate, iron(III) nitrate-9-hydrate, iron(III) acetylacetonate and iron(III)-sulfate-5-hydrate are suitable.

In one embodiment, the reaction for the preparation of compound (6) is performed in an organic solvent.

In one embodiment, the organic solvent is tetrahydrofuran, ethyl acetate, methanol or ethanol. Ethanol is preferred.

In one embodiment, the compound alkyl 4-(5-amino-1-methyl-*1H*-benzimidazol-2-yl)butanoate (6) is alkylated to alkyl 4-[5-[bis(2-hydroxyethyl)amino]-1-methyl-*1H-*benzimidazol-2-yl]butanoate (7, CAS No. 3543-74-6 (R=ethyl-)), wherein R is CH₃ (methyl-), C₂H₅ (ethyl-), C₃H₇ (propyl-) or C₄H₉ (butyl-), generally CₙH₂ₙ₊₁.

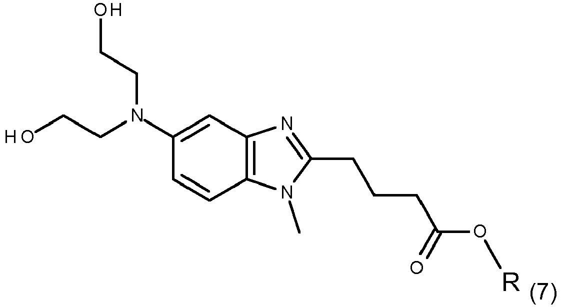

In one embodiment, the alkylation is performed with ethylene oxide or a 2-haloethanol.

In one embodiment, the 2-haloethanol is 2-chloroethanol. In one embodiment, the 2-haloethanol is 2-bromoethanol. In one embodiment, the 2-haloethanol is 2-iodoethanol.

In one embodiment, an alkali metal- or earth alkaline metal iodide, in particular sodium or potassium iodide, is added to accelerate the reaction. Thereby, the reaction time is shortened by approx. 30% through the use of 2-bromoethanol and potassium iodide.

In one embodiment, the reaction for the preparation of alkyl 4-[5-[bis(2-hydroxyethyl)amino]-1-methyl-*1H*-benzimidazol-2-yl]butanoate (7) is performed in water, a C1- to C4-alkyl alcohol or a mixture of water and alcohol.

In one embodiment, the alcohol is selected from methanol, ethanol, 1-propanol, 2-propanol, 1-butanol, 2-methyl-1-propanol, 2-butanol or 2-methyl propan-2-ol.

In one embodiment, the alkylation is performed at pH values <9.1, preferably at pH values between 4 and 8, in particular at pH values between 4.0 and 5.5.

In one embodiment, the alkylation is performed in water.

In one embodiment, the alkylation is performed at a temperature of 50°C to 80°C, preferably at 60°C to 70°C.

In one embodiment, the pH value is controlled by the addition of salt and/or buffer solutions of acetic acid / alkali metal acetate, ammonium- or alkali metal hydrogen carbonates, ammonium-, alkali metal or alkaline earth metal carbonate, ammonium-, mono-, di- or trimethylammonium acetate, mono-, di- or triethylammonium acetate, ammonium propionate, disodium hydrogenphosphate, dipotassium hydrogenphosphate, diammonium hydrogenphosphate alone or in combination with commercial "ammonium carbonate" (1:1 mixture of ammonium hydrogen carbonate and ammonium carbonate), ammonium hydrogen carbonate or general salts of a weak to medium strength acid (citric acid, tartaric acid, succinic acid, malonic acid or oxalic acid) in the form of the ammonium-, alkylammonium-, alkali metal or alkali earth metal salts.

In one embodiment, the alkylation step is performed with 2-bromoethanol in water by the addition of ammonium acetate at the outset of the reaction at pH 4 to 6 without addition of other organic or inorganic bases.

In one embodiment, the pH value of this system for the alkylation during the main reaction lies between pH 4.2 to 5.5. Towards the end of the reaction the pH value can or may be lowered to 4 without instigating noticeable ester cleavage. Lower pH values and raised temperatures (> approx. 75°C) effect clear ester cleavage and a sharp reduction of yield and quality.

By performing the method of the invention with addition of the stated salts individually or in combination, an otherwise unavoidable over-alkylation is markedly reduced. This over-alkylation arises quickly and in increasing quantities at pH values > 6.5. This is also the case with all procedures described hitherto involving the use of organic or inorganic bases, as these are already added at the outset of the reaction. The yield dramatically drops as the proportion of over-alkylated product increases.

In one embodiment, the alkylation is performed with a molar ratio of > 8 mol 2-haloethanol (2-chloroethanol, 2-bromoethanol or 2-iodoethanol) to 1 mol alkyl 4-(5-amino-1-methyl-*1H-*benzimidazol-2-yl)butanoate (6, CAS No 3543-73-5 (R=ethyl-)). It is advantageous thereby to utilise, for example, 10 mol 2-bromethanol. Thereby, an otherwise significant side reaction which forms a dimeric compound such as compound 7C is strongly suppressed. In comparison to all other by-products, this dimeric compound cannot be removed through recrystallisation.

In one embodiment, the pH of the alkylation reaction is controlled through addition of salt and/or buffer solutions, wherein a molar ratio of > 2 mol of salt- and/or buffer solution to 1 mol compound (6) is used.

In one embodiment, a molar ratio of 3 mol ammonium acetate to 1 mol compound (6) is used.

In one embodiment, the alkylation is performed with 2-bromoethanol with a molar ration of > 8 mol bromoethanol to 1 mol alkyl 4-(5-amino-1-methyl-*1H*-benzimidazol-2-yl)butanoate (6, CAS No 3543-73-5 (R=ethyl-), whereby particularly the formation of a dimer of compound (7) such as compound (7C) is supressed, particularly below a content of 0.15%, and with addition of an alkali metal- or earth alkaline metal iodide, in particular sodium or potassium iodide, whereby particularly the alkylation is accelerated, particularly to 70% of the reaction time without addition.

In one embodiment, compound (7) is chlorinated to alkyl 4-[5-[bis(2-chloroethyl)amino]-1-methyl-*1H*-benzimidazol-2-yl]butanoate (8, CAS No. 87475-54-5 (R=ethyl-)) with a suitable chlorinating agent, wherein R is CH₃ (methyl-), C₂H₅ (ethyl-), C₃H₇ (propyl-) or C₄H₉ (butyl-), generally CₙH₂ₙ₊₁.

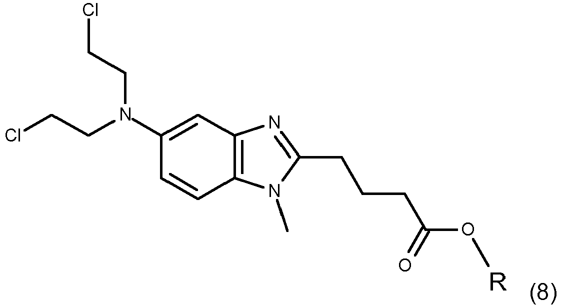

In one embodiment, the chlorinating agent is thionyl chloride (SOCl₂), sulfuryl chloride (SO₂Cl₂), phosphorus oxychloride (POCl₃), phosphorus trichloride (PCl₃) or phosphorus pentachloride (PCl₅). Thionyl chloride (SOCl₂) is a preferred chlorinating agent.

In one embodiment of this aspect of the invention, the reaction is performed in a chlorinated hydrocarbon solvent, in particular dichloromethane or trichloromethane.

In another embodiment, the reaction is carried out in aromatic solvents, in particular in toluene or chlorobenzene.

In one embodiment, product (8) exists as a water-soluble hydrochloride or analogous salt thereof.

In one embodiment, compound (8) is hydrolysed to 4-[5-[bis(2-chloroethyl)amino]-1-methyl-*1H-*benzimidazol-2-yl]butanoic acid (9, Bendamustine, CAS-Nr. 16506-27-7).

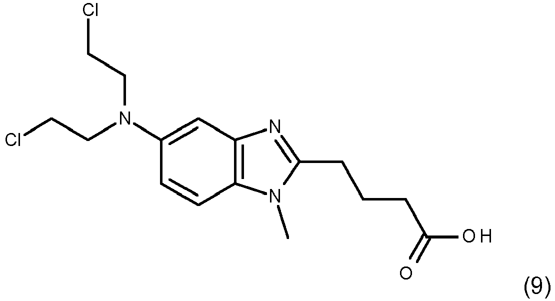

In one embodiment, the hydrolysis of compound (8) is performed with aqueous hydrochloric acid.

In one embodiment, product (9) is available as water-soluble hydrochloride (CAS-Nr. 3543-75-7) or as an analogous salt thereof.

Wherever reference is made herein to an embodiment of the invention, and such an embodiment refers to one feature of the invention only, it is intended that such an embodiment may be combined with any other embodiment which refers to a different feature.

The invention is further illustrated, without limitations, by the following figures and examples, from which further features, advantages or embodiments can be derived. The examples do not limit but illustrate the invention.

### Description of the Figures:

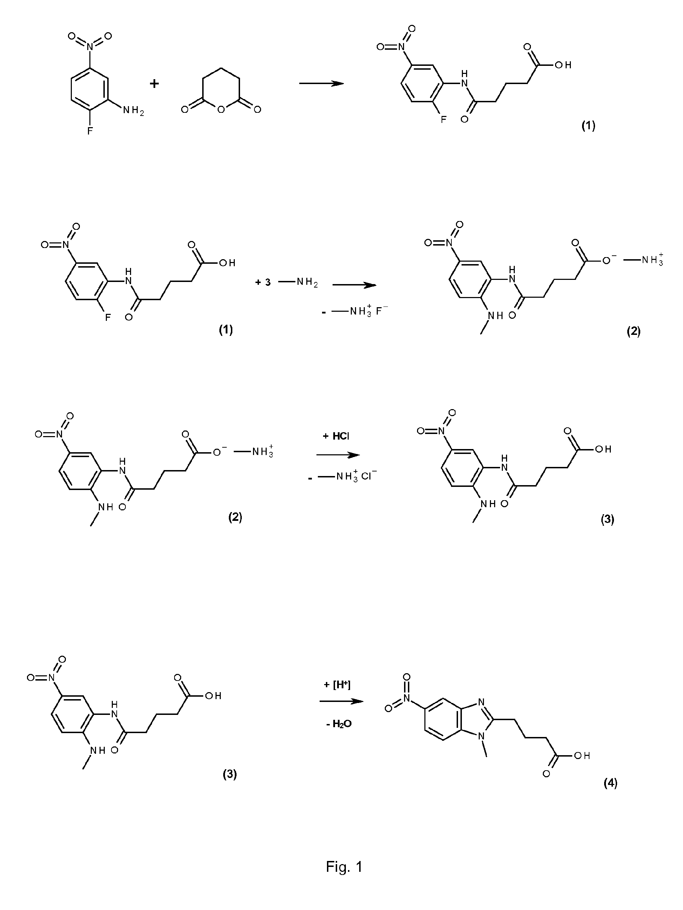

- Fig. 1: shows the reaction sequence for the preparation of 4-(1-methyl-5-nitro-*1H-*benzimidazol-2-yl)butanoic acid (4), starting from 2-fluoro-5-nitroaniline via the intermediates 5-(2-fluoro-5-nitroanilino)-5-oxopentanoic acid (1), methylammonium 5-[2-(methylamino)-5-nitroanilino]-5-oxopentanoate (2) and 5-[2-(methylamino)-5-nitroanilino]-5-oxopentanoic acid (3).

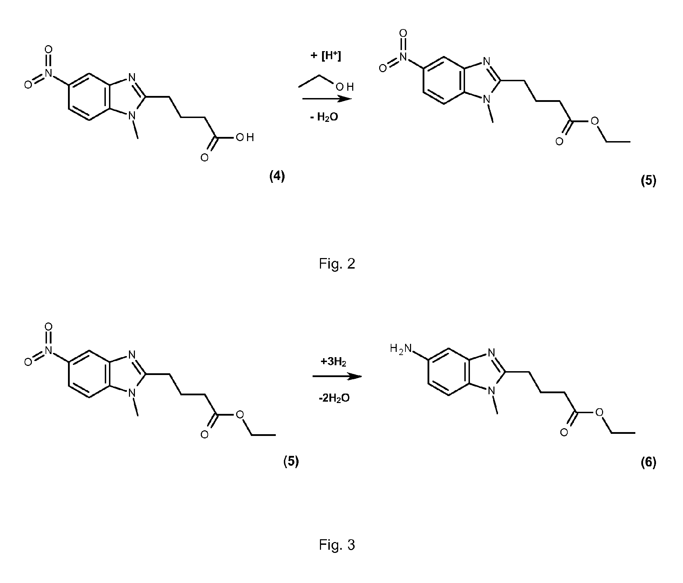

- Fig. 2: shows the esterification of 4-(1-methyl-5-nitro-*1H-*benzimidazol-2-yl)butanoic acid (4) to ethyl 4-(1-methyl-5-nitro-*1H*-benzimidazol-2-yl)butanoate (5).
- Fig. 3: shows the hydrogenation of ethyl 4-(1-methyl-5-nitro-*1H*-benzimidazol-2-yl)butanoate (5) to ethyl 4-(5-amino-1-methyl-*1H*-benzimidazol-2-yl)butanoate (6).

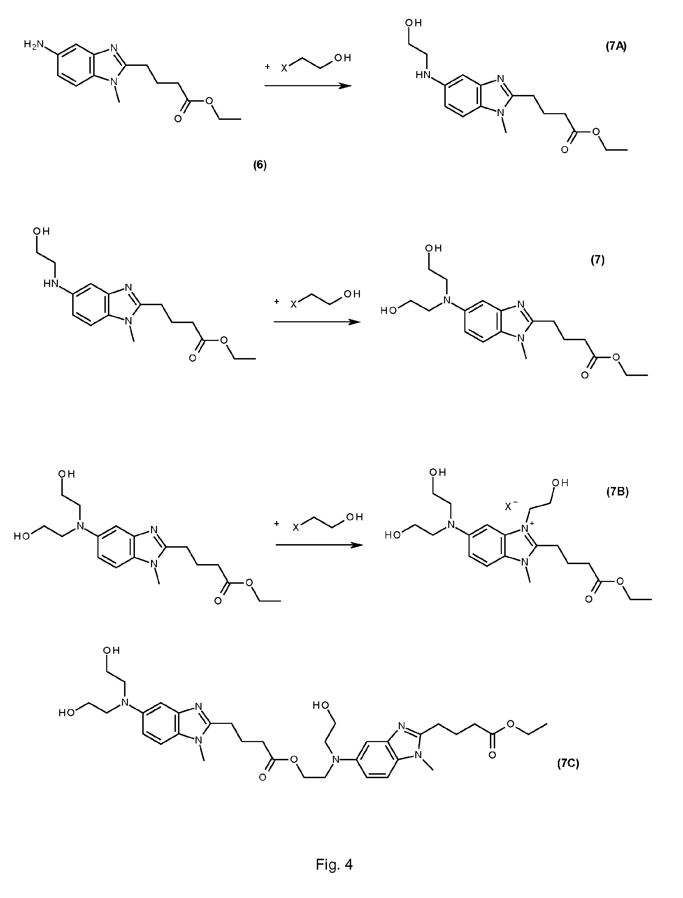

- Fig. 4: shows the alkylation of ethyl 4-(5-amino-1-methyl-*1H*-benzimidazol-2-yl)butanoate (6) to ethyl 4-[5-[bis(2-hydroxyethyl)amino]-1-methyl-*1H*-benzimidazol-2-yl]butanoate (7) with the intermediate ethyl 4-[5-(2-hydroxyethylamino-1-methyl-*1H-*benzimidazole-2-yl]butanoate (7A) as well as the undesired over-alkylation product 6-[bis-(2-hydroxyethyl)-amino]-2-(3-ethoxycarbonyl-propyl)-1-(2-hydroxyethyl)-3-methyl-3H-benzimidazol-1-iumhalogenide (7B) and the undesired dimer (7C) of compound (7).

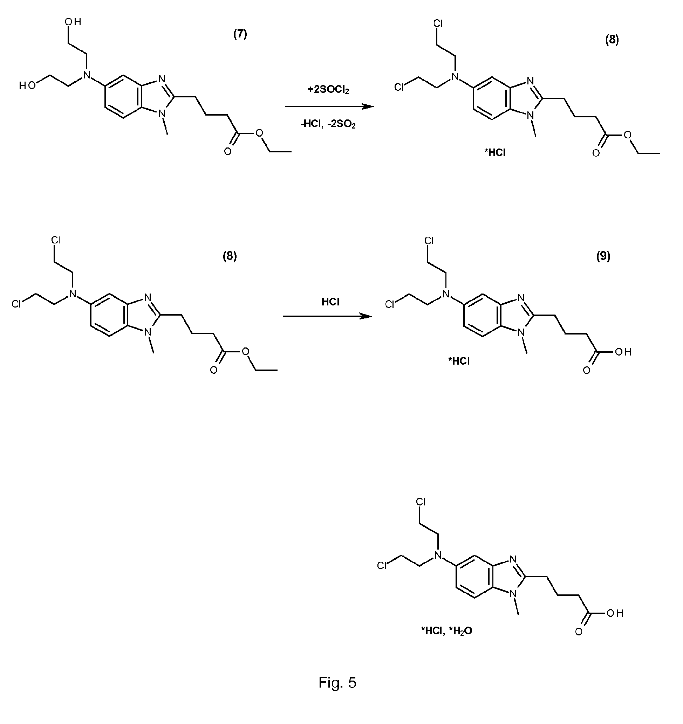

- Fig. 5: shows the chlorination and subsequent hydrolysis of ethyl 4-[5-[bis(2-hydroxyethyl)amino]-1-methyl-*1H-*benzimidazol-2-yl]butanoate (7) to 4-[5-[bis(2-chloroethyl)amino]-1-methyl-*1H*-benzimidazol-2-yl]butanoic acid (9, Bendamustine) via the intermediate ethyl 4-[5-[bis(2-chloroethyl)amino]-1-methyl-*1H*-benzimidazol-2-yl]butanoate (8).

### Examples

### Example 1: Synthesis of 4-(1-methyl-5-nitro-1H-benzimidazol-2-yl)butanoic acid (4)

95 g (608.5 mmol) of 2-fluoro-5-nitroaniline were dissolved together with 83.1 g (691.9 mmol) of glutaric anhydride (95 %) in 490 g of toluene. Subsequently, the mixture was heated at ca. 85°C for 4 h and the resulting crystal mush concentrated to dryness at up to 70°C under vacuum.

To this dried crystal mush were added 284 g of 40 % methylamine aqueous solution and 94 g water. The residue dissolved quickly and the mixture began to boil after a short time (15-25 minutes). The solution was held at 75 °C (to limit consumption of methylamine) for approximately 3 h and then diluted with 966 g of water and 680 g of propan-2-ol. 156 g of 37 % hydrochloric acid were added with stirring at 55 - 60 °C to neutralize the reaction mixture. Compound (4) was subsequently precipitated through further addition of 37 % hydrochloric acid. The precipitate was filtered, washed with water and dried.

The yield of compound (4) was 157 g (596.4. mmol) with a content of > 99 %. (98.0 % of theory).

Instead of 40% methylamine aqueous solution, also a 25% methylamine alcoholic solution, for example a methylamine methanol or ethanol solution, can be used.

### Example 2: Synthesis of ethyl 4-(1-methyl-5-nitro-1H-benzimidazol-2-yl)butanoate (5)

157 g (596.4 mmol) of compound (4) were suspended in 1374 g of ethanol, 43.8 g of 96 % sulfuric acid were added and the reaction mixture heated for approx. 10 h under reflux before concentrating to the crystallization concentration. The catalytic acid was preferably neutralized with triethylamine, the crystallization initiated by cooling and then the precipitate filtered. Compound (5) was washed with ethanol and subsequently dried.

The yield of compound (5) was 163 g (559.5 mmol) with a content of > 99 % (93.8 % of theory).

### Example 3: Synthesis of ethyl 4-(5-amino-1-methyl-1H-benzimidazol-2-yl)butanoate (6)

163 g (559.5 mmol) of compound (5) were dissolved in 1875 g ethanol. 14 g of palladium catalyst on activated carbon doped with iron (5% Pd, 1% Fe) and an additional 0.8 g of iron(II) sulfate 7-hydrate or 0.8 g of iron(III) nitrate 9-hydrate were added. Compound (5) was then hydrogenated at a hydrogen pressure of up to 4 bar until complete conversion of the starting compound (5).

The catalyst was removed by filtration, and the ethanolic solution concentrated until a dry product remained. This residue was crystallized from propan-2-ol or ethyl acetate.

The yield of compound (6) was 128 g (489,8 mmol) with a content of > 99 % (87.5 % of theory).

The overall yield of compound (6) was 80.4% of theory in relation to 2-fluoro-5-nitroaniline. In comparison, the synthesis according to DD34727 starting from N-1-methyl-4-nitrobenzene-1,2-diamine (CAS 41939-61-1) is characterized by a yield of 39.0%.

### Example 4: Synthesis of ethyl 4-[5-[bis(2-hydroxyethyl)amino]-1-methyl-1H-benzimidazol-2-yl]butanoate (7)

To a solution of 81.3 g (650.6 mmol) 2-bromoethanol, 1 g potassium iodide and 100 g water was added 17.0 g (65 mmol) compound (6). The reaction mixture was heated to 65-70 °C and held at this temperature for 8 h to 12 h. The pH value of the solution was held between 4.2-5.5 during this period by dropwise addition of a solution of 20.0 g (151.4 mmol) diammonium hydrogen phosphate in 35 g water. The control of pH over the duration of the reaction was effected through use of a pH electrode. The conversion was followed by HPLC. The reaction was continued until the fraction of compound (7A) was ≤ 1.5 %. Thereby ca. 8% of compound (7B) had formed and the proportion of compound (7) was ca. 87%. The reaction mixture was subsequently concentrated to dryness at ca. 55-60 °C under vacuum. To the residue was added 150 g water and, preferably with an alkali metal carbonate, the pH value adjusted to ca. 8.5. The desired product (7) was extracted with 200 g methylene chloride or 225 g chloroform, and the organic phase subsequently washed with 60 - 80 g water. The organic phase was then concentrated to dryness and the remaining oil or already crystalline residue dissolved in 200 g ethyl acetate or alternatively in 60 g actetonitrile. Compound (7) crystallised at ca. 5 °C and was filtered under suction, washed with 20 g cold ethyl acetate or alternatively with 15 g cold acetonitrile and dried at 60 -70 °C. The yield of compound (7) was 18.3 g (52.4 mmol) with a content of ≥ 98.2% (80.5 % of theory). The crude product contained ≤0.6% compound (7A) and compound (7B) respectively as well as <0.15% of compound (7C).

The crude product obtained was recrystallized from ethyl acetate, or alternatively from acetonitrile, toluene, propan-2-ol, tetrahydrofuran, acetone, isopropyl acetate or water, prior to further conversion to compound (8). Thereby the yield of compound (7) was 17.2 g (94.0% recrystallization yield) with a content of > 99.2%, wherein compound (7A) was removed below a content of 0.2 % and compound (7B) below 0.3 %. Through the course of the reaction, the content of compound (7C) was kept below 0.15 %, as this compound can only poorly be removed by recrystallization from the above described solvents. The overall yield of this step was 76.5% of theory and was thus ca. 12.5% higher than that described in the procedure using ethylene oxide as according to DD34727 and ca. 31% higher in comparison to the favoured procedure of WO2011079193 involving addition of Hünig's base.

### Example 5: Synthesis of ethyl 4-[5-[bis(2-hydroxyethyl)amino]-1-methyl-1H-benzimidazol-2-yl]butanoate (7)

Analogous to Example 4 but with use of 6.9 g (65 mmol) sodium carbonate dissolved in 25 g water to hold the pH value between 4.2 - 5.5. Identical results in terms of yield and quality.

### Example 6: Synthesis of ethyl 4-[5-[bis(2-hydroxyethyl)amino]-1-methyl-1H-benzimidazol-2-yl]butanoate (7)

Analogous to Example 4 but with use of 9.0 g (65 mmol) potassium carbonate dissolved in 12 g water to hold the pH value between 4.2 - 5.5. Identical results in terms of yield and quality.

### Example 7: Synthesis of ethyl 4-[5-[bis(2-hydroxyethyl)aminol-1-methyl-1H-benzimidazol-2-yl]butanoate (7)

Analogous to Example 4 but with use of 19 g (109.1 mmol) dipotassium hydrogen phosphate dissolved in 25 g water to hold the pH value between 4.2 - 5.5. Identical results in terms of yield and quality.

### Example 8: Synthesis of ethyl 4-[5-[bis(2-hydroxyethyl)aminol-1-methyl-1H-benzimidazol-2-yl]butanoate (7)

This example is a scaled-up analogue of Example 6 with use of 340 g (1.3 mol) compound (6), 2000 ml water and 1625 g (13 mol) 2-bromoethanol. The reaction was performed without potassium iodide at 69 - 70°C.. The pH value was held between 4.2 - 5.5 using a solution of 138 g sodium carbonate (1.3 mol) in 500 g water. Until a content of compound (7A) of ≤1.5% was reached, the duration of the reaction was 13.5 h. The yield of compound (7) was 365 g crude and 343.5 g after recrystallisation from acetonitrile (75.6% of theory).

### Example 9: Synthesis of 4-[5-[bis(2-chloroethyl)amino]-1-methyl-1H-benzimidazol-2-yl]butanoic acid (9, Bendamustine hydrochloride hydrate)

250 g (0.7154 mol) compound (7) was dissolved in 2000 ml methylene chloride and 212 g (1.78 mol) thionyl chloride added at -1 °C within a period of 30 minutes. Thereby the temperature rose briefly to ca. 4 °C. Following addition the reaction solution was stirred for a further 30 minutes at -1 °C. The solution was then stirred for ca. 16 h at ca. 22 °C. Thereafter the solvent and excess thionyl chloride were removed by distillation under vacuum. To hydrolyse the ester the remaining residue (compound 8 as its hydrochloride) was treated with 2.6 kg 37% hydrochloric acid and 1.4 I water, heated to ca. 75 °C and held at this temperature for 30 -40 minutes. 25 g activated carbon was then added and stirred for 10 minutes at 75 °C. The solution was filtered and concentrated under vacuum. To crystallise crude compound (9) the residue was dissolved in 1000 ml water at ca. 55 °C, the solution cooled to ca. -2 °C and then held at this temperature for ca. 30 minutes. The crude product (9) was filtered off, washed with 250 g water and 200 g acetone and dried for 2 h at ca. 35 °C under vacuum.

The yield of crude compound (9) was 245 g (0.5936 mol) and had a water content of 4.5 % (83% of theory).

245 g compound (9) were dissolved in 330 g 37% hydrochloric acid at ca. 40 °C, treated with 1.28 kg water (temperature ca. 35 °C) and 650 g acetone (temperature ca. 35 °C) and stirred for 10 minutes. Crystallisation was initiated by the addition of 0.5 g Bendamustine hydrochloride hydrate, the mixture cooled within a period of 2 h to ca. -20 °C and then held at this temperature for ca. 90 minutes.

The precipitate was filtered under suction. The crystals were washed initially with a mixture of 120 g water and 90 g acetone and subsequently with 275 g acetone.

The pure Bendamustine hydrochloride hydrate was dried for ca. 2 h at ca. 35°C under vacuum. Thus 225 g (0.545 mol) of pure (> 99.8% content) compound (9) was obtained with an overall yield of 76.2% for this step.

Through drying of the Bendamustine hydrochloride hydrate under vacuum at ca. 50 °C the water content could be adjusted to ca. 1 % in contrast to 4.4% of the mono hydrates.

## Claims

1. A method for the preparation of 4-(1-methyl-5-nitro-*1H*-benzimidazol-2-yl)butanoic acid (4), from 2-fluoro-5-nitroaniline, comprising the steps:
a) conversion of 2-fluoro-5-nitroaniline to 5-(2-fluoro-5-nitroanilino)-5-oxopentanoic acid (1) using glutaric anhydride; 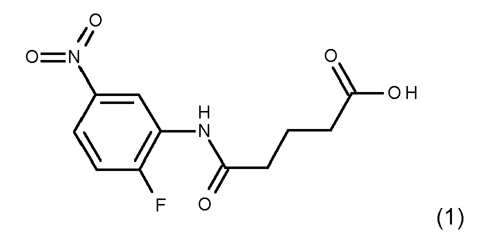
b) conversion of the reaction product (1) of step a) to methylammonium 5-[2-(methylamino)-5-nitroanilino]-5-oxopentanoate (2) using methylamine; 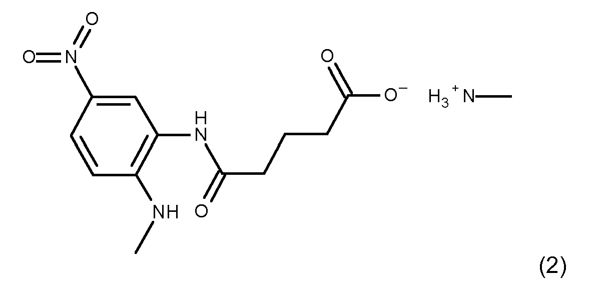
c) conversion of the reaction product (2) of step b) to 5-[2-(methylamino)-5-nitroanilino]-5-oxopentanoic acid (3) and 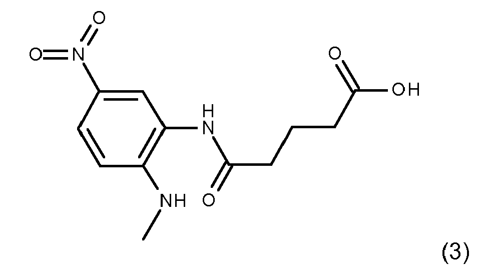
d) condensation of the reaction product (3) of step c) to 4-(1-methyl-5-nitro-*1H-*benzimidazol-2-yl)butanoic acid (4):

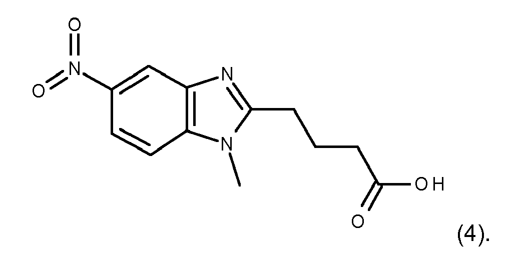

2. A method according to claim 1, wherein steps a) to d) are performed in a single process step without isolation of the reaction products.

3. A method according to any one of the previous claims, wherein tetrahydrofuran or toluene is used as solvent.

4. A method according to any one of the previous claims, **characterized in that** the product of step d), 4-(1-methyl-5-nitro-*1H*-benzimidazol-2-yl)butanoic acid (4), is converted in a step e) with a C1- to C4- alkyl alcohol to alkyl 4-(1-methyl-5-nitro*1H*--benzimidazol-2-yl)butanoate (5) in the presence of a strong acid, wherein R is CH₃ (methyl-), C₂H₅ (ethyl-), C₃H₇ (propyl-) or C₄H₉ (butyl-).

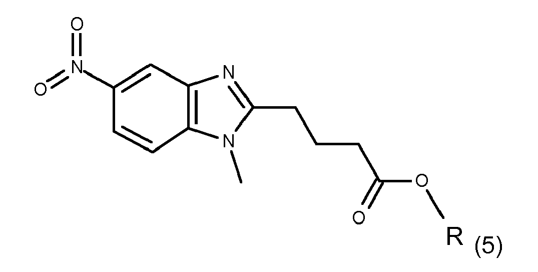

5. A method according to claim 4, **characterized in that** the amount of said strong acid in step e) is from 10 - 90 % in relation to the amount of substance of the product of step d) 4-(1-methyl-5-nitro-*1H*-benzimidazol-2-yl)butanoic acid (4).

6. A method according to any one of the previous claims, wherein steps a) to e) are performed in a single process step without isolation of the reaction products.

7. A method according to any one of the claims 4 to 6, **characterized in that** the reaction product alkyl 4-(1-methyl-5-nitro-*1H*-benzimidazol-2-yl)butanoate (5) is converted to alkyl 4-(5-amino-1-methyl-*1H*-benzimidazol-2-yl)butanoate (6) through selective reduction of the nitro group, wherein R is CH₃ (methyl-), C₂H₅ (ethyl-), C₃H₇ (propyl-) or C₄H₉ (butyl-).

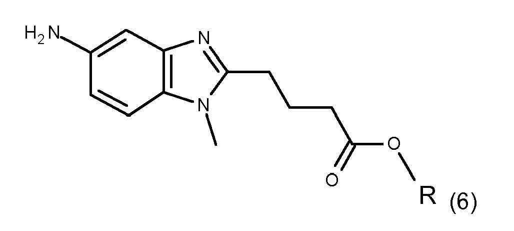

8. A method according to claim 7, **characterized in that** said reduction is performed with hydrogen using an iron-doped palladium catalyst.

9. A method according to claim 8, **characterized in that** an iron(II) or iron(III) salt of an organic or inorganic acid is added to said iron-doped palladium catalyst.

10. A method according to any one of the claims 7 to 9, **characterized in that** the reaction product alkyl 4-(5-amino-1-methyl-*1H*-benzimidazol-2-yl)butanoate (6) is alkylated to alkyl 4-[5-[bis(2-hydroxyethyl)amino]-1-methyl-*1H*-benzimidazol-2-yl]butanoate (7), wherein R is CH₃ (methyl-), C₂H₅ (ethyl-), C₃H₇ (propyl-) or C₄H₉ (butyl-).

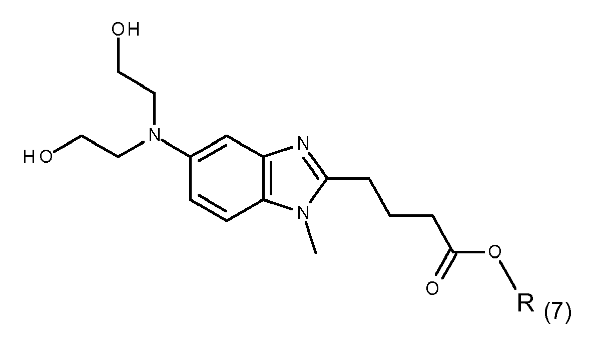

11. A method according to claim 10, **characterized in that** the alkylation is performed with a 2-haloethanol at a molar ratio of greater than 8 mol 2-haloethanol per 1 mol alkyl 4-(5-amino-1-methyl-*1H*-benzimidazol-2-yl)butanoate (6).

12. A method according to claim 10 or 11, **characterized in that** the reaction is performed at a pH <9.1, preferably at pH 4 to 6, even more preferably at pH 4.2 to 5.5.

13. A method according to any one of the claims 10 to12, **characterized in that** the reaction product alkyl 4-[5-[bis(2-hydroxyethyl)amino]-1-methyl-*1H*-benzimidazol-2-yl]butanoate (7) is converted to alkyl 4-[5-[bis(2-chloroethyl)amino]-1-methyl-*1H*-benzimidazol-2-yl]butanoate (8) with a chlorinating agent, wherein R is CH₃ (methyl-), C₂H₅ (ethyl-), C₃H₇ (propyl-) or C₄H₉ (butyl-).

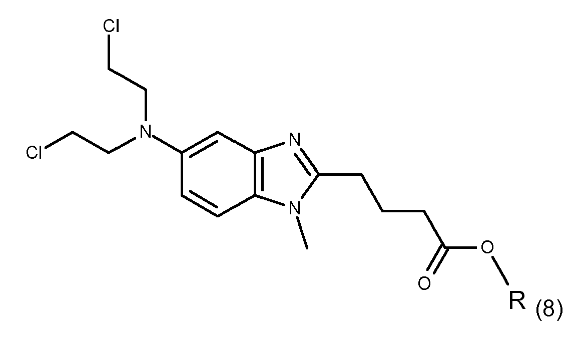

14. A method according to claim 13, **characterized in that** said chlorinating agent is thionyl chloride.

15. A method according to claim 13 or 14, **characterized in that** alkyl 4-[5-[bis(2-chloroethyl)amino]-1-methyl-*1H*-benzimidazol-2-yl]butanoate (8) is hydrolysed to 4-[5-[bis(2-chloroethyl)amino]-1-methyl-*1H*-benzimidazol-2-yl]butanoic acid (9, Bendamustine) or the hydrochloride thereof.

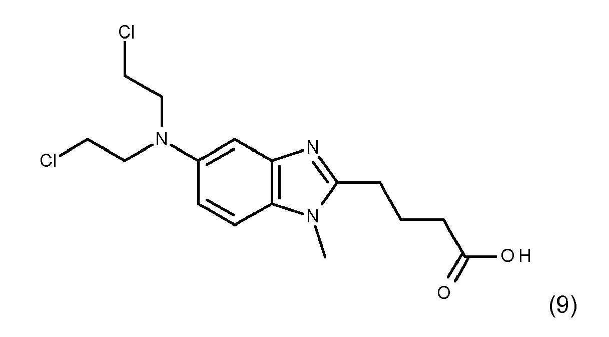

## Patentansprüche

1. Verfahren zur Herstellung von 4-(1-Methyl-5-nitro-*1H*-benzimidazol-2-yl)butansäure (4) ausgehend von 2-Fluor-5-nitroanilin umfassend die Schritte:
a) Umsetzung von 2-Fluor-5-nitroanilin mit Glutarsäureanhydrid zu 5-(2-Fluor-5-nitroannilin)-5-oxopentansäure (1); 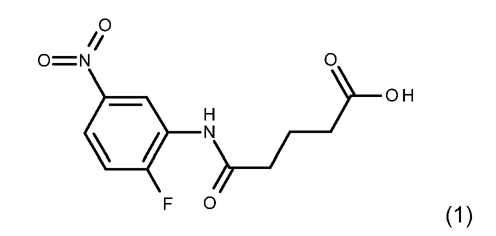
b) Umsetzung des Reaktionsprodukts (1) des Schrittes a) mit Methylamin zu Methylammonium-5-(2-methylamino-5-nitroannilin)-5-oxopentanoat (2); 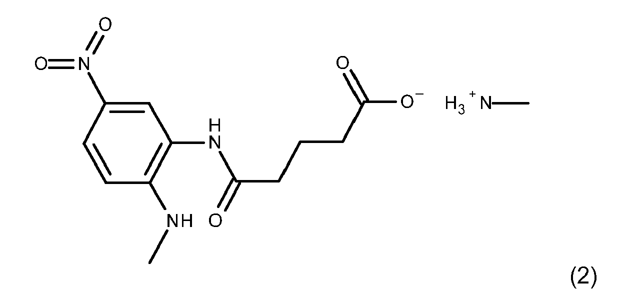
c) Umsetzung des Reaktionsprodukts (2) des Schrittes b) zu 5-(2-Methylamino-5-nitroannilin)-5-oxopentansäure (3) und 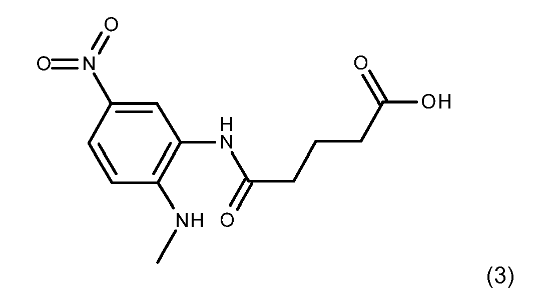
d) Kondensation des Reaktionsproduktes (3) des Schrittes c) zu 4-(1-Methyl-5-nitro-1H-benzimidazol-2-yl)butansäure (4).

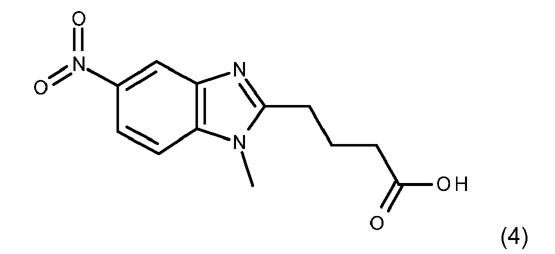

2. Verfahren gemäß Anspruch 1, wobei die Schritte a) bis d) in einem Verfahrensschritt ohne Isolation der Reaktionsprodukte durchgeführt werden.

3. Verfahren gemäß einem der vorherigen Ansprüche, wobei als Lösungsmittel Tetrahydrofuran oder Toluol verwendet wird.

4. Verfahren gemäß einem der vorherigen Ansprüche, **dadurch gekennzeichnet, dass** das Produkt des Schrittes d) 4-(1-Methyl-5-nitro-*1H*-benzimidazol-2-yl)butansäure (4) in einem Schritt e) mit einem C1-C4-Alkylakohol zu 4-(1-Methyl-5-nitro-*1H*-benzimidazol-2-yl)butansäurealkylester (5) in Gegenwart einer starken Säure umgesetzt wird, wobei R CH₃ (methyl-), C₂H₅ (ethyl-), C₃H₇ (propyl-) oder C₄H₉ (butyl-) ist.

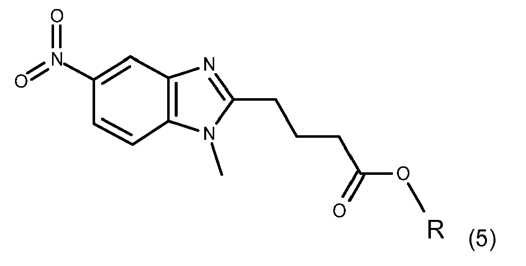

5. Verfahren gemäß Anspruch 4, **dadurch gekennzeichnet, dass** die Menge der starken Säure in Schritt e) 10 - 90 % der Stoffmenge des eingesetzten Produktes des Schrittes d) 4-(1-Methyl-5-nitro-*1H*-benzimidazol-2-yl)butansäure (4) beträgt.

6. Verfahren gemäß einem der vorherigen Ansprüche, wobei die Schritte a) bis e) in einem Verfahrensschritt ohne Isolation der Reaktionsprodukte durchgeführt werden.

7. Verfahren gemäß einem der Ansprüche 4 bis 6, **dadurch gekennzeichnet, dass** das Reaktionsprodukt 4-(1-Methyl-5-nitro-*1H*-benzimidazol-2-yl)butansäurealkylester (5) durch selektive Reduktion der Nitrogruppe zu 4-(1-Methyl-5-amino-*1H*-benzimidazol-2-yl)butansäurealkylester (6) umgesetzt wird, wobei R CH₃ (methyl-), C₂H₅ (ethyl-), C₃H₇ (propyl-) oder C₄H₉ (butyl-) ist.

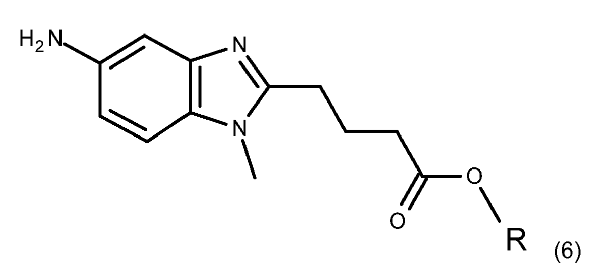

8. Verfahren gemäß Anspruch 7, wobei die Reaktion mit Wasserstoff unter Verwendung von mit Eisen dotierten Palladiumkatalysatoren durchgeführt wird.

9. Verfahren gemäß Anspruch 8, wobei ein Eisen(II)- oder Eisen(III)-salz einer anorganischen oder organischen Säure dem verwendeten Palladiumkatalysator zugesetzt wird.

10. Verfahren gemäß einem der Ansprüche 7 bis 9, **dadurch gekennzeichnet, dass** das Reaktionsprodukt 4-(1-Methyl-5-amino-*1H*-benzimidazol-2-yl)butansäurealkylester (6) zu 4-{5-[Bis-(2-hydroxyethyl)amino]-1-methyl-*1H*-benzimidazol-2-yl}butansäurealkylester (7) alkyliert wird, wobei R CH₃ (methyl-), C₂H₅ (ethyl-), C₃H₇ (propyl-) oder C₄H₉ (butyl-) ist.

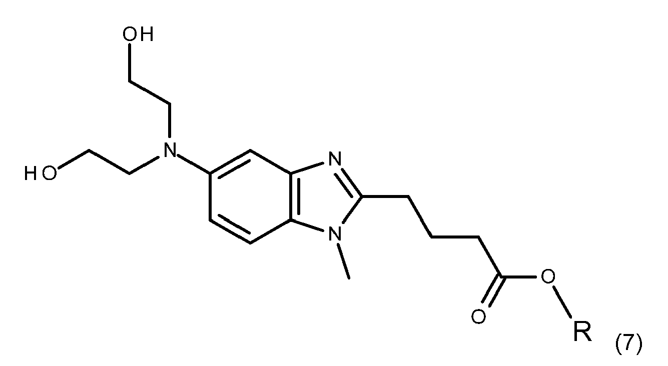

11. Verfahren gemäß Anspruch 10, **dadurch gekennzeichnet, dass** die Alkylierung mit einem 2-Haloethanol bei einem Molverhältnis vom größer als 8 mol 2-Haloethanol pro 1 mol 4-(1-Methyl-5-amino-*1H*-benzimidazol-2-yl)butansäurealkylester (6) durchgeführt wird.

12. Verfahren gemäß Anspruch 10 oder 11, **dadurch gekennzeichnet, dass** die Reaktion bei pH<9,1, bevorzugt bei pH 4 bis pH 6, weiter bevorzugt bei pH 4,2 bis 5,5, durchgeführt wird.

13. Verfahren gemäß einem der Ansprüche 10 bis 12, **dadurch gekennzeichnet, dass** das Reaktionsprodukt 4-{5-[Bis-(2-hydroxyethyl)amino]-1-methyl-*1H*-benzimidazol-2-yl}butansäurealkylester (7) mit einem Chlorierungsmittel zu 4-{5-[Bis-(2-chlorethyl)amino]-1-methyl-*1H*-benzimidazol-2-yl}butansäurealkylester (8) umgesetzt wird, wobei R CH₃ (methyl-), C₂H₅ (ethyl-), C₃H₇ (propyl-) oder C₄H₉ (butyl-) ist.

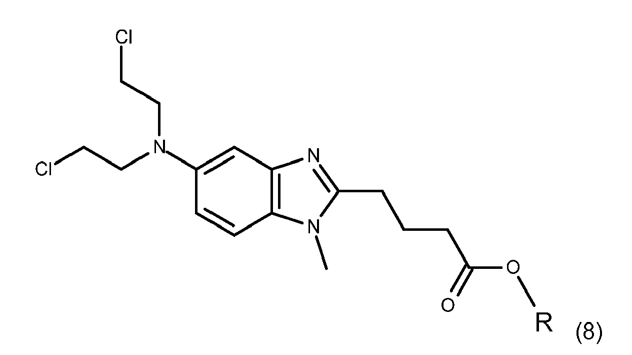

14. Verfahren gemäß Anspruch 13, **dadurch gekennzeichnet, dass** das Chlorierungsmittel Thionylchlorid ist.

15. Verfahren gemäß Anspruch 13 oder 14, **dadurch gekennzeichnet, dass** 4-{5-[Bis-(2-chlorethyl)amino]-1-methyl-*1H*-benzimidazol-2-yl}butansäurealkylester (8) zu 4-{5-[Bis-(2-chlorethyl)amino]-1-methyl-*1H*-benzimidazol-2-yl}butansäure (9, Bendamustin) oder dessen Hydrochlorid hydrolysiert wird.

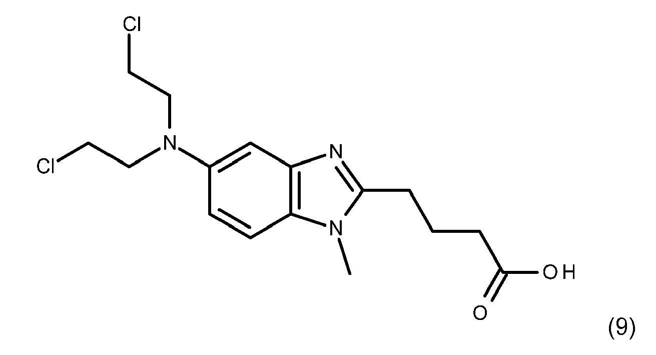

## Revendications

1. Méthode de préparation de l'acide 4-(1-méthyl-5-nitro-1H-benzimidazol-2-yl)-butanoïque (4) à partir de la 2-fluoro-5-nitroaniline, comprenant les étapes de :
a) conversion de la 2-fluoro-5-nitroaniline en acide 5-(2-fluoro-5-nitroanilino)-5-oxopentanoïque (1) avec de l'anhydride glutarique ; 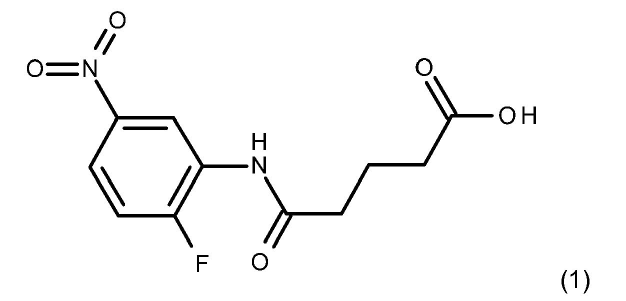
b) conversion du produit de réaction (1) de l'étape a) en 5-[2-(méthylamino)-5-nitroanilino]-5-oxopentanoate de méthylammonium (2) avec de la méthylamine ; 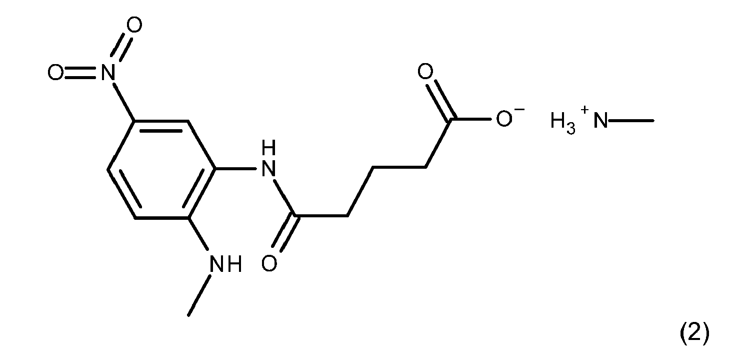
c) conversion du produit de réaction (2) de l'étape b) en acide 5-[2-(méthylamino)-5-nitroanilino]-5-oxopentanoïque (3) et 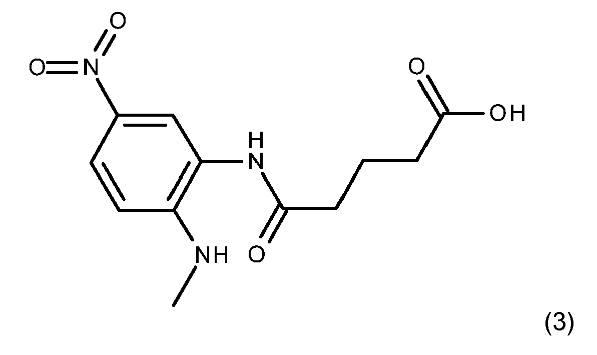
d) condensation du produit de réaction (3) de l'étape c) en acide 4-(1-méthyl-5-nitro-1 H-benzimidazol-2-yl)-butanoïque (4) :

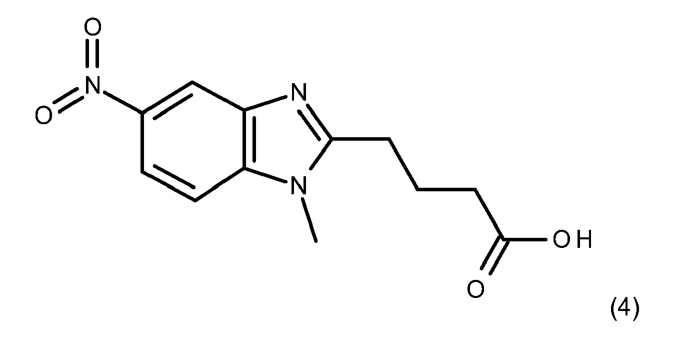

2. Méthode selon la revendication 1 où les étapes a) à d) sont effectuées en une unique étape de procédé sans isolation des produits réactionnels.

3. Méthode selon l'une des revendications précédentes, où on utilise le tétrahydrofurane ou le toluène comme solvant.

4. Méthode selon l'une des revendications précédentes, **caractérisée en ce que** le produit de l'étape d), acide 4-(1-méthyl-5-nitro-1H-benzimidazol-2-yl)-butanoïque (4), est converti en une étape e) avec un alcool en C1 à C4 en butanoate de 4-(1-méthyl-5-nitro-1H-benzimidazol-2-yl) d'alkyle (5) en présence d'un acide fort, où R est CH₃ (méthyle), C₂H₅ (éthyle), C₃H₇ (propyle) ou C₄H₉ (butyle).

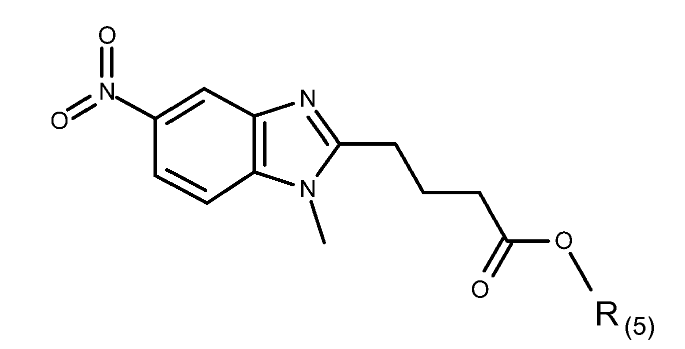

5. Méthode selon la revendication 4, **caractérisée en ce que** la quantité dudit acide fort à l'étape e) est entre 10 et 90 % par rapport à la quantité de substance du produit de l'étape d) acide 4-(1-méthyl-5-nitro-1 H-benzimidazol-2-yl)-butanoïque (4).

6. Méthode selon l'une quelconque des revendications précédentes, où les étapes a) à e) sont effectuées en une unique étape de procédé sans isolation des produits réactionnels.

7. Méthode selon l'une quelconque des revendications 4 à 6, **caractérisée en ce que** le produit réactionnel butanoate de 4-(1-méthyl-5-nitro-1H-benzimidazol-2-yl) d'alkyle (5) est converti en butanoate de 4-(5-amino-1-méthyl-1H-benzimidazol-2-yl) d'alkyle (6) par réduction sélective du groupe nitro, où R est CH₃ (méthyle), C₂H₅ (éthyle), C₃H₇ (propyle) ou C₄H₉ (butyle).

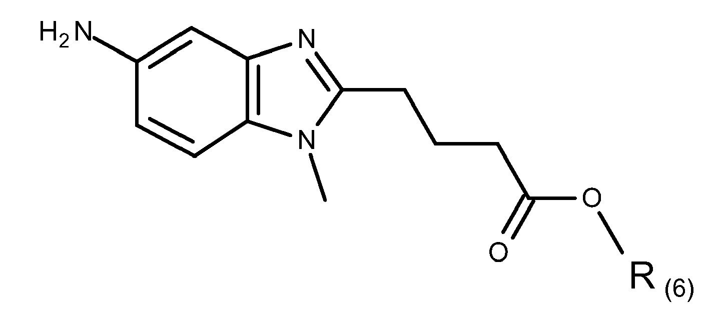

8. Méthode selon la revendication 7, **caractérisée en ce que** ladite réduction est effectuée avec de l'hydrogène et un catalyseur palladium dopé au fer.

9. Méthode selon la revendication 8, **caractérisée en ce qu'**un sel de fer (II) ou de fer (III) d'un acide organique ou minéral est ajouté audit catalyseur palladium dopé au fer.

10. Méthode selon l'une quelconque des revendications 7 à 9, **caractérisée en ce que** le produit de réaction butanoate de 4-(5-amino-1-méthyl-1 H-benzimidazol-2-yl) d'alkyle (6) est alkylé en butanoate de 4-(5-[bis-(2-hydroxyéthyl)-amino]-1-méthyl-1H-benzimidazol-2-yl) d'alkyle (7) où R est CH₃ (méthyle), C₂H₅ (éthyle), C₃H₇ (propyle) ou C₄H₉ (butyle).

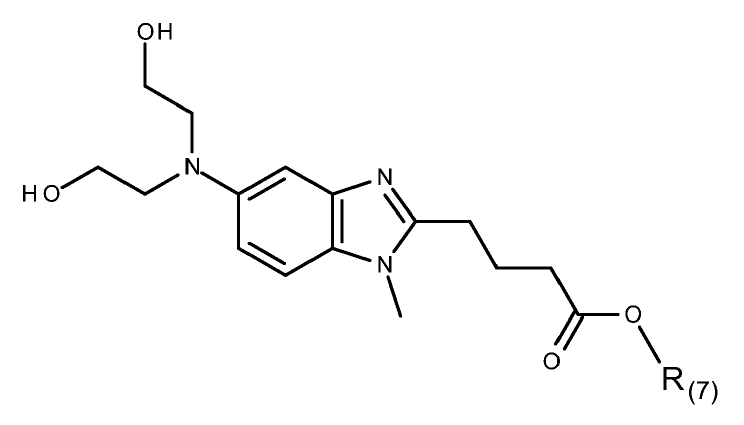

11. Méthode selon la revendication 10, **caractérisée en ce que** l'alkylation est effectuée avec un 2-haloéthanol à un rapport molaire supérieur à 8 moles de 2-haloéthanol par mole de 4-(5-amino-1-méthyl-1H-benzimidazol-2-yl) d'alkyle (6).

12. Méthode selon la revendication 10 ou 11, **caractérisée en ce que** la réaction est effectuée à pH < 9,1, préférentiellement à pH entre 4 et 6, plus préférentiellement à pH entre 4,2 et 5,5.

13. Méthode selon l'une quelconque des revendications 10 à 12, **caractérisée en ce que** le produit réactionnel butanoate de 4-(5-[bis-(2-hydroxyéthyl)-amino]-1-méthyl-1H-benzimidazol-2-yl) d'alkyle (7) est converti en butanoate de 4-(5-[bis-(2-chloroéthyl)-amino]- 1-méthyl-1 H-benzimidazol-2-yl) d'alkyle (8) par un agent chlorant, où R est CH₃ (méthyle), C₂H₅ (éthyle), C₃H₇ (propyle) ou C₄H₉ (butyle).

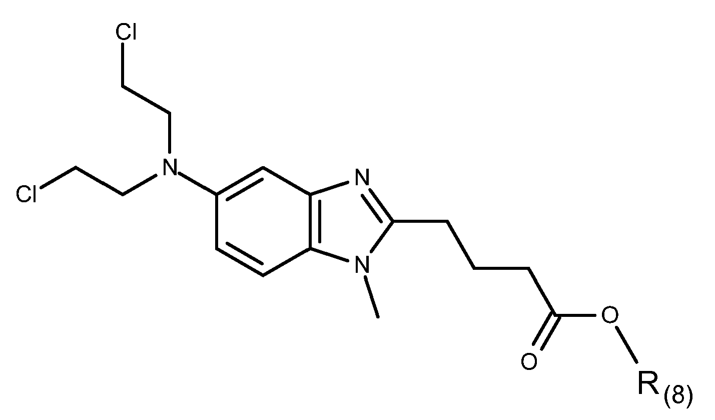

14. Méthode selon la revendication 13, **caractérisée en ce que** ledit agent chlorant est du chlorure de thionyle.

15. Méthode selon la revendication 13 ou 14, **caractérisée en ce que** le butanoate de 4-(5-[bis-(2-chloroéthyl)-amino]-1-méthyl-1H-benzimidazol-2-yl) d'alkyle (8) est hydrolysé en acide 4-(5-[bis-(2-chloroéthyl)-amino]-1-méthyl-1H-benzimidazol-2-yl) butanoïque (9, bendamustine) ou en son chlorhydrate.
